# EUROPEAN PATENT APPLICATION

(11) **EP 2 283 864 A1**
(43) Date of publication of application: **16.02.2011**
(21) Application number: 10010944.6
(22) Date of filing: 16.07.2003
(51) Int. Cl.: A61K 47/02, A61K 47/10, A61K 47/26, A61K 9/10, A61K 9/14, A61K 31/192, A61K 31/58

(54) **Liquid dosage compositions fo stable nanoparticulate active agents**

(30) Priority: 16.07.2002 US 396530 P
(62) Divisional of application: 03764723.7
(71) Applicant: Elan Pharma International Ltd., County Clare (IE)
(72) Inventor: Bosch, H. William, Bryn Mawr, PA 19010 (US); Hilborn, Matthew R., Spring City PA 19475 (US); Hovey, Douglas C., Gilbertsville PA 19525 (US); Kline, Laura J., Harleysville, PA 19438 (US); Lee, Robert W., Boyertown, PA 19512 (US); Pruitt, John D., Collegeville, PA 19426 (US); Ryde, Niels P., Malvern, PA 19355 (US); Ryde, Tuula A., Malvern, PA 19355 (US); Xu, Shuqian, Sunnyvale, CA 94086 (US)
(74) Representative: Wichmann, Hendrik

(57) **Abstract**

The present invention relates to liquid dosage compositions of stable nanoparticulate active agents. The liquid dosage compositions of the invention include osmotically active crystal growth inhibitors that stabilize the nanoparticulate active agents against crystal and particle size growth of the active agent.

## Description

### FIELD OF THE INVENTION

The present invention relates to liquid dosage compositions of stable nanoparticulate active agents. The liquid dosage compositions of the invention comprise at least one osmotically active crystal growth inhibitor, and preferably a crystal growth inhibitor that does not solubilize the nanoparticulate active agent present in the composition.

### BACKGROUND OF THE INVENTION

### I. Background Regarding Nanoparticulate Compositions

Nanoparticulate active agent compositions, first described in U.S. Patent No. 5,145,684 ("the '684 patent"), are particles consisting of a poorly soluble therapeutic or diagnostic agent having adsorbed onto, or associated with, the surface thereof a non-crosslinked surface stabilizer. The '684 patent does not teach liquid dosage compositions of nanoparticulate active agents comprising an osmotically active crystal growth inhibitor.

Many factors can affect active agent bioavailability, including the dosage form and various properties, *e.g*., dissolution rate of the active agent. Poor bioavailability is a significant problem encountered in the development of pharmaceutical compositions, particularly those containing an active agent that is poorly soluble in water. By decreasing the particle size of an active agent, the surface area of the composition is increased, thereby generally resulting in an increased bioavailability.

Methods of making nanoparticulate active agent compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate active agent compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill;" 6,432,381 for "Methods for Targeting Drug Delivery to the Upper and/or Lower Gastrointestinal Tract," and 6,592,903 for "Nanoparticulate Dispersions Comprising a Synergistic Combination of a Polymeric Surface Stabilizer and Dioctyl Sodium Sulfosuccinate," all of which are specifically incorporated by reference. In addition, U.S. Patent Application No. 20020012675 A1, published on January 31, 2002, for "Controlled Release Nanoparticulate Compositions," describes nanoparticulate compositions, and is specifically incorporated by reference. None of these references describe liquid dosage compositions of nanoparticulate active agents comprising an osmotically active crystal growth inhibitors.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

### II. Background Regarding Liquid Dosage Compositions

Liquid dosage compositions are useful in a variety of therapies and routes of administration. Unlike solid dosage forms, active agent particles present in liquid dosage compositions must remain as discrete, well-dispersed particles in the suspending media for longer periods of time than for solid dosage forms. Such liquid dosage compositions of nanoparticulate active agents must be physically stable; that is the active agent particles must not aggregate together, as well as not increase in fundamental particle size.

Nanoparticulate active agent particles present in liquid dosage compositions, especially dilute liquid dosage compositions, can be unstable, *i.e*., prone to crystal growth. It is believed that the solubilization and subsequent re-crystallization of the component active agent particles generates the crystals. This process results in large crystal formation over a period of time in the nanoparticulate active agent composition. In addition, some nanoparticulate active agent compositions exhibit active agent particle aggregation over a period of time. Although such crystal growth and particle aggregation are often insignificant under normal conditions, under certain circumstances substantial crystal growth and particle aggregation can occur.

Crystal growth and particle aggregation in nanoparticulate active agent compositions are highly undesirable for several reasons. Such crystal growth can be observed by light microscopy and can also be detected in light scattering measurements. Large crystals in the nanoparticulate active agent composition may cause increased toxic effects of the active agent, especially when the preparation is in an injectable formulation. This is also true for active agent particle aggregation, as injectable formulations preferably have an effective average particle size of no greater than about 250 nm.

In addition, for oral formulations, the presence of large crystals and/or particle aggregation, and therefore varying particle sizes, can create a variable bioavailability profile because smaller particles dissolve faster than the larger aggregates or larger crystal particles. For active agents having a dissolution-rate limited bioavailability, a faster rate of dissolution is associated with greater bioavailability and a slower rate of dissolution is associated with a lower bioavailability. In such cases, bioavailability is related to the surface area of an administered active agent and, therefore, bioavailability increases with a reduction in the particle size of the dispersed active agent. With a composition having widely varying particle sizes, bioavailability becomes highly variable and inconsistent and dosage determinations become difficult.

Moreover, because such crystal growth and particle aggregation are uncontrollable and unpredictable, the quality of the nanoparticulate active agent compositions is inconsistent. Finally, the mere occurrence of crystal growth indicates that the nanoparticulate active agent compositions is not a "stable" pharmaceutical formulation, because such crystal growth indicates that the nanoparticulate active agent particles are continually solubilizing and re-crystallizing. This may in turn cause degradation of the active agent with numerous undesirable ramifications.

Nanoparticulate active agent formulations generally require the presence of a surface stabilizer to prevent active agent particle aggregation, as described in U.S. Patent No. 5,145,684. However, certain nanoparticulate active agent formulations can be susceptible to active agent particle aggregation even when a surface stabilizer is present, such as when the formulation is heated to temperatures above the cloud point of the surface stabilizer, or after the formulation has been lyophilized.

Several methods have been suggested in the prior art for preventing active agent particle aggregation following heat sterilization, including adding a cloud point modifier to the nanoparticulate active agent composition and purifying the surface stabilizer. For example, U.S. Patent No. 5,298,262 describes the use of an anionic or cationic cloud point modifier in nanoparticulate active agent compositions and U.S. Patent No. 5,346,702 describes nanoparticulate active agent compositions having a nonionic surface stabilizer and a non-ionic cloud point modifier. The cloud point modifier enables heat sterilization of the nanoparticulate active agent compositions with low resultant active agent particle aggregation. U.S. Patent No. 5,470,583 describes nanoparticulate active agent compositions having a non-ionic surface stabilizer and a charged phospholipid as a cloud point modifier.

All of these various prior art methods share one common feature: they require an additional substance added to the nanoparticulate active agent formulation to inhibit or prevent aggregation of the nanoparticulate active agent composition. The addition of such a substance can be detrimental as it may induce adverse effects, particularly for injectable formulations. Moreover, cloud point modifiers are often highly toxic, especially when administered via the intravenous route. Thus, this minimizes the usefulness of such substances in pharmaceutical compositions.

In addition, U.S. Patent No. 5,302,401 describes nanoparticulate active agent compositions having polyvinylpyrrolidone (PVP) as a surface stabilizer and sucrose as a cryoprotectant (allowing the nanoparticulate active agent to be lyophilized). The compositions exhibit minimal particle aggregation following lyophilization.

Another method of limiting aggregation of nanoparticulate active agent compositions during sterilization known prior to the present invention was the use of purified surface stabilizers. U.S. Patent No. 5,352,459 describes nanoparticulate active agent compositions having a purified surface stabilizer (having less than 15% impurities) and a cloud point modifier. However, purification of surface stabilizers can be expensive and time consuming, thus significantly raising production costs of compositions requiring such stabilizers to produce a stable nanoparticulate active agent composition.

U.S. Patent No. 6,267,989 is directed to the surprising discovery that nanoparticulate active agent compositions having an optimal effective average particle size exhibit minimal active agent particle aggregation and crystal growth, even following prolonged storage periods or exposure to elevated temperatures. However, it sometimes can be difficult and costly to achieve such a specified particle size.

In contrast to active agent particle aggregation, the nanoparticulate active agent particles of some liquid dosage compositions are prone to increases in fundamental particle size, *e.g*., crystal growth, especially when stored for extended periods of time or at elevated temperatures. Certain complex crystal growth inhibitors for nanoparticulate active agent compositions are described in U.S. Patent Nos. 5,665,331, 5,565,188, 5,834,025, 5,747,001, 5,718,919, and 6,264,922. The inhibitors taught by these patents are chemical compounds that have at least 75% of the compound, on a molecular basis, structurally identical to the pharmaceutical agent. The use of complex crystal growth inhibitors as described above is undesirable because it requires a chemically unique crystal growth modifier to be newly synthesized for every active agent that is to be made into a nanoparticulate active agent formulation. In many cases the chemical synthesis may be difficult or expensive, and the toxicological and pharmacological effects of each and every new crystal growth modifier must be evaluated before the compound can be safely incorporated in a pharmaceutical dosage form.

It would be desirable to provide liquid dosage compositions of nanoparticulate active agent in which the active agent is stabilized against crystal growth, and which do not require the synthesis and incorporation of new chemical entities. Such novel liquid dosage compositions, utilizing known pharmaceutical ingredients as crystal growth inhibitors, are especially convenient in pharmaceuticals and diagnostics. The present invention satisfies this need.

### SUMMARY OF THE INVENTION

The present invention is directed to liquid dosage compositions of stable nanoparticulate active agents comprising particles of at least one active agent having an effective average particle size of less than about 2000 nm, at least one surface stabilizer, and at least one osmotically active crystal growth inhibitor. In particular, the invention is directed to the surprising discovery that commonly used, non-toxic pharmaceutical ingredients which are osmotically active, such as glycerol, mannitol, and sodium chloride, can function as crystal growth inhibitors in liquid dosage compositions of nanoparticulate active agent.

The present invention also includes a method of making a liquid dosage composition of stable nanoparticulate active agents comprising contacting particles of at least one active agent with at least one surface stabilizer for a time and under conditions sufficient to provide a nanoparticulate active agent composition having an effective average particle size of less than about 2000 nm. Either before, during, or after active agent particle size reduction, at least one osmotically active crystal growth inhibitor is added to the active agent composition.

The present invention also includes a method of treating a subject with a liquid dosage form of at least one stable nanoparticulate active agent comprising administering to the subject an effective amount of a liquid dosage composition according to the invention. The liquid dosage composition is particularly useful in treating patient populations such as the elderly, infants, and pediatrics.

### DETAILED DESCRIPTION OF THE INVENTION

The present invention relates to liquid dosage compositions of stable nanoparticulate active agents comprising: (1) at least one nanoparticulate active agent having an effective average particle size of less than about 2000 nm; (2) at least one surface stabilizer adsorbed to or associated with the surface of the active agent; and (3) at least one osmotically active crystal growth inhibitor in the liquid dosage form. The crystal growth inhibitor is either partially, substantially, or completely dissolved in the liquid media of the composition.

Benefits of the liquid dosage compositions of the invention include, but are not limited to: (1) decreased toxicity of the active agent as a result of decreased crystal growth, particularly for injectable liquid dosage compositions; (2) decreased particle aggregation; (3) a more consistent bioavailability profile, aiding in dosage determination, due to the more consistent active agent particle sizes present in the liquid dosage composition; (4) more consistent quality of the liquid dosage compositions; (5) increased stability of the liquid dosage compositions, due to the stability of the active agent particle sizes; (6) increased chemical stability of the active agent; (7) the liquid dosage compositions do not require the addition of potentially toxic cloud point modifiers; (8) the liquid dosage compositions do not require purification of the one or more surface stabilizers; (9) the liquid dosage compositions do not require the active agent to be present in a narrowly defined particle size (such as that required by U.S. Patent No. 6,267,989); and (10) the liquid dosage compositions do not require the synthesis of chemically unique crystal growth modifiers.

In addition, as compared to liquid dosage compositions of a microparticulate or solubilized form of the same active agent, present at the same dosage, the liquid dosage compositions of nanoparticulate active agents of the present invention may provide one or more of the following benefits: (1) lower viscosity; (2) better patient compliance due to the perception of a lighter formulation which is easier to consume and digest; (3) ease and accuracy of dispensing due to low viscosity; (4) avoidance of organic solvents or pH extremes; (5) longer active agent dose retention in blood and tumors for some active agents; (6) more rapid absorption of active agents; (7) liquid dosage compositions suitable for parenteral administration; (8) the liquid dosage compositions can be sterile filtered; (9) increased bioavailability; (10) smaller dosage volume; (11) smaller doses of active agent required to obtain the same pharmacological effect; (12) higher dose loading; (13) improved pharmacokinetic profiles; (14) substantially similar and/or bioequivalent pharmacokinetic profiles of the nanoparticulate active agent compositions when administered in the fed versus the fasted state; and (15) bioadhesive liquid dosage compositions of nanoparticulate active agents.

The present invention is described herein using several definitions, as set forth below and throughout the application.

"About" will be understood by persons of ordinary skill in the art and will vary to some extent on the context in which the term is used. If there are uses of the term which are not clear to persons of ordinary skill in the art given the context in which it is used, "about" will mean up to plus or minus 10% of the particular term.

"Conventional" or "non-nanoparticulate active agent" shall mean an active agent which is solubilized or which has an effective average particle size of greater than about 2 microns.

"Osmotically active" as used herein with respect to a crystal growth inhibitor shall mean that the crystal growth inhibitor is soluble in the liquid media of the invention and is present as solubilized molecules or ions.

As used herein, the term "particle size" refers to the equivalent spherical diameter of a particle having a certain volume. Specifically, the volume of a theoretically spherical particle of a drug can be defined by: Volume (V)=(4/3)πr³. Therefore, the theoretical diameter can be defined by: Diameter (D)=2• (3V/4π)^{1/3}. Similarly, the surface area of a particle can also be determined from the diameter of the theoretically spherical particle by the equation: Surface Area (SA) = 4π(0.5D)². When used in the context of particle distributions, "particle size" refers to the mean diameter of the distribution calculated on the basis of volume or weight statistics. As used herein, volume and weight particle size measurements are interchangeable.

"Poorly water soluble active agents" as used herein means active agents having a solubility in water of less than about 30 mg/ml, preferably less than about 20 mg/ml, preferably less than about 10 mg/ml, or preferably less than about 1 mg/ml, at ambient temperature. Such active agents tend to be eliminated from the gastrointestinal tract before being absorbed into the circulation. Moreover, poorly water soluble active agents tend to be unsafe for intravenous administration techniques, which are used primarily in conjunction with highly water soluble drug substances.

As used herein with reference to stable active agent particles, "stable" includes, but is not limited to, one or more of the following parameters: (1) that the active agent particles do not appreciably flocculate or agglomerate due to interparticle attractive forces, or otherwise significantly increase in particle size over time; (2) that the physical structure of the active agent particles is not altered over time, such as by conversion from an amorphous phase to crystalline phase; (3) that the active agent particles are chemically stable; and/or (4) where the active agent has not been subject to a heating step at or above the melting point of the active agent in the preparation of the nanoparticles of the invention.

"Therapeutically effective amount" as used herein with respect to an active agent dosage shall mean a dosage that provides the specific pharmacological response for which the active agent is administered in a significant number of subjects in need of such treatment. It is emphasized that "therapeutically effective amount," administered to a particular subject in a particular instance will not always be effective in treating the diseases described herein, even though such dosage is deemed a 'therapeutically effective amount' by those skilled in the art. It is to be further understood that active agent dosages are, in particular instances, measured as oral dosages, or with reference to active agent levels as measured in blood.

### II. Preferred Characteristics of the Liquid Dosage Compositions of the Invention

### A. Decreased Toxicity

The liquid dosage compositions of the invention may provide a decreased level of toxicity as compared to prior liquid dosage compositions of the same nanoparticulate active agent as well as prior liquid dosage compositions of a microparticulate or solubilized form of the same active agent, present at the same dosage. This is because the nanoparticulate active agents present in liquid dosage compositions can be prone to crystal growth and particle aggregation over a period of time. Large crystals and particle aggregation in the nanoparticulate active agent composition may cause increased toxic effects of the active ingredient, especially when the preparation is in an injectable formulation.

### B. More Consistent and Improved Bioavailability

The liquid dosage compositions of the invention may provide a more consistent bioavailability profile, which aids in dosage determination. Specifically, a liquid dosage composition having highly variable active agent particle sizes, including large crystals, can result in a variable bioavailability profile from dose to dose because smaller particles dissolve faster than the larger aggregates or larger crystal particles. For active agents having a dissolution-rate limited bioavailability, such as poorly water soluble active agents, a faster rate of dissolution is associated with greater bioavailability and a slower rate of dissolution is associated with a lower bioavailability. In such cases, bioavailability is related to the surface area of an administered active agent and, therefore, bioavailability increases with a reduction in the particle size of the dispersed agent. With a composition having widely varying particle sizes, bioavailability becomes highly variable and inconsistent and dosage determinations become difficult. This can be particularly problematic for active agents having a narrow preferred dosage range, such as immunosuppressants, chemotherapy agents, etc.

The liquid dosage compositions of nanoparticulate active agents of the invention preferably exhibit increased bioavailability, at the same dose of the same active agent, require smaller doses, and show longer plasma half-life as compared to prior conventional active agent formulations.

In another aspect of the invention, the liquid dosage compositions of nanoparticulate active agents of the invention may have enhanced bioavailability such that the active agent dosage can be reduced as compared to a conventional non-nanoparticulate liquid dosage form of the same active agent, resulting in a decrease in toxicity associated with such active agents.

### C. Decreased Dosage Volume and Increased Dose Loading

Greater bioavailability of the liquid dosage compositions of nanoparticulate active agents of the invention can enable a smaller solid dosage volume. This is particularly significant for patient populations such as the elderly, juvenile, and infant.

The liquid dosage compositions of the invention can be formulated for dosages in any volume, but are preferably formulated into equivalent or smaller volumes than existing conventional liquid dosage compositions of the same active agent (*i.e*., non-nanoparticulate or solubilized active agent formulations). For example, the invention encompasses liquid dosage compositions formulated into a volume which is at least half that of an existing conventional liquid dosage form of the same active agent. Even smaller dosage volumes are also possible..

The maximal dose loading of the liquid dosage compositions of the invention is significantly higher than the maximal dose loading provided by conventional prepared formulations of the same active agents. A dose loading which is double or more than that utilized in conventional liquid dosage compositions of the same active agent is expected to be useful.

### D. Increased Stability of the Liquid Dosage Compositions

Because crystal growth and particle aggregation in prior art liquid dosage compositions of nanoparticulate active agents can be uncontrollable and unpredictable, the quality of the nanoparticulate active agent compositions is inconsistent. The mere occurrence of crystal growth indicates that the nanoparticulate active agent formulation is not a "stable" pharmaceutical formulation, because such crystal growth indicates that the nanoparticulate active agent particles are continually solubilizing and re-crystallizing.

Moreover, such solubilizing and re-crystallizing of an active agent can result in chemical degradation of the active agent. This is highly undesirable as chemical degradation of an active agent almost always leads to a loss or significant decrease in the desired activity of the active agent. In addition, the by-products of such degradation may be toxic.

### E. Decreased Viscosity of the Liquid Dosage Compositions of the Invention

The liquid dosage compositions of the present invention may also exhibit reduced viscosity as compared to prior art liquid dosage compositions of the same active agent, present at the same dosage. In the present invention, the liquid dosage compositions can have a low viscosity and, preferably, the viscosity demonstrates Newtonian behavior. Typically, the nanoparticulate active agents are produced in a size range where it is believed Brownian motion keeps the particles suspended, obviating the use of thickening agents and additives to prevent settling or caking. Thus, an especially preferred embodiment is one in which no thickening or flocculating agents are required to render the liquid dosage composition stable.

Another important aspect of the invention is that the liquid dosage composition may be "water-like" and "silky." As such, a preferred embodiment of the invention comprises a liquid dosage composition that is substantially less gritty than a conventional non-nanoparticulate liquid dosage composition of the same active agent. "Gritty," as used herein refers to the property of particulate matter that can be seen with the naked eye or that which can be felt as "gritty." The liquid dosage compositions of the invention can be poured out of or extracted from a container as easily as water, whereas a conventional (*i.e*., non-nanoparticulate or solubilized active agent) liquid dosage composition of the same active agent, present at the same dose loading, is notably more "sluggish".

It is desirable to have a liquid dosage composition for oral administration that is palatable, silky in texture, and which has a low viscosity at high dose loading levels. Such water-like formulations can result in increased patient compliance because the formulation is more agreeable to consume as compared to a large solid dose form ("horse pill") or highly viscous liquid dosage form. These properties are especially important when considering juvenile patients, terminally ill patients, and patients suffering from gastrointestinal tract dysfunction or other conditions where nausea and vomiting are symptoms. For example, patients suffering from cancer or AIDS-related complications are commonly hypermetabolic and, at various stages of disease, exhibit gastrointestinal dysfunction. Additionally, drugs used to treat these conditions often cause nausea and vomiting. Viscous or gritty formulations, and those that require a relatively large dosage volume, are not well tolerated by patient populations suffering from wasting associated with these diseases because the formulations can exacerbate nausea and encourage vomiting.

Highly viscous and turbid solutions are also difficult to accurately dispense. Viscous solutions can be difficult to pour, especially if the product is refrigerated.

Liquid dosage compositions having low viscosity and small active agent particle size are desirable for parenteral administration. Viscous solutions can be problematic in parenteral administration because such solutions require a slow syringe push and can stick to tubing. Further, it is unsafe to administer intravenous formulations that have a particle size greater than about 2000 nm**.** Moreover, conventional formulations of poorly water-soluble active agents tend to be unsafe for intravenous administration techniques, which are used primarily in conjunction with highly water-soluble substances.

Viscosity is concentration and temperature dependent. Typically, a higher concentration results in a higher viscosity, while a higher temperature results in a lower viscosity. Viscosity as defined herein refers to a measurements taken at about 20°C. (The viscosity of water at 20°C is 1 mPa·s.) The invention encompasses equivalent viscosities measured at different temperatures.

Typically the viscosity of the liquid dosage compositions of the invention, at a shear rate of 0.1 (1/s), can be from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, or from about 5 mPa·s to about 1 mPa·s.

The viscosity of the liquid dosage compositions of the invention preferably can be less than the viscosity of a standard or conventional liquid dosage form of the same active agent, at about the same concentration of active agent. Preferably the viscosity of the liquid dosage compositions of the invention can be less than about 1/200, less than about 1/100, less than about 1/50, less than about 1/25, or less than about 1/10 of the viscosity of a conventional liquid dosage compositions of the same active agent, at about the same concentration per ml of the active agent.

In other embodiments of the invention, preferably the viscosity of the liquid dosage compositions of the invention is less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, or less than about 90% of the viscosity of a standard conventional liquid dosage form of the same active agent at about the same concentration per ml of active agent.

The invention also provides low viscosity liquid dosage compositions of nanoparticulate active agents that do not require thickening agents.

### F. Sterile Filtration of the Liquid Dosage Compositions of the Invention

Low viscosity liquid dosage compositions of nanoparticulate active agents can be sterile filtered, obviating the need for heat sterilization, which can harm or degrade many active agents as well as result in crystal growth and particle aggregation. Sterile filtration can be difficult because of the required small particle size of the composition. Filtration is an effective method for sterilizing homogeneous solutions when the membrane filter pore size is less than or equal to about 0.2 microns (200 nm) because a 0.2 micron filter is sufficient to remove essentially all bacteria. Sterile filtration is normally not used to sterilize conventional suspensions of micron-sized active agents because the active agent particles are too large to pass through the membrane pores.

A sterile liquid dosage form is particularly useful in treating immunocompromised patients, infants or juvenile patients, and the elderly, as these patient groups are the most susceptible to infection caused by a non-sterile liquid dosage form.

### G. Improved Pharmacokinetic Profiles

The invention also preferably provides liquid dosage compositions of nanoparticulate active agents having a desirable pharmacokinetic profile when administered to mammalian subjects. The desirable pharmacokinetic profile of the liquid dosage compositions preferably includes, but is not limited to: (1) that the Tₘₐₓ of an active agent when assayed in the plasma of a mammalian subject following administration is preferably less than the Tₘₐₓ for a conventional, non-nanoparticulate form of the same active agent, administered at the same dosage; (2) that the Cₘₐₓ of an active agent when assayed in the plasma of a mammalian subject following administration is preferably greater than the Cₘₐₓ for a conventional, non-nanoparticulate form of the same active agent, administered at the same dosage; and/or (3) that the AUC of an active agent when assayed in the plasma of a mammalian subject following administration, is preferably greater than the AUC for a conventional, non-nanoparticulate form of the same active agent, administered at the same dosage.

The desirable pharmacokinetic profile, as used herein, is the pharmacokinetic profile measured after the initial dose of an active agent. The compositions can be formulated in any way as described herein and as known to those of skill in the art.

A preferred liquid dosage composition of the invention exhibits in comparative pharmacokinetic testing with a non-nanoparticulate formulation of the same active agent, administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, or not greater than about 10% of the Tₘₐₓ, exhibited by the non-nanoparticulate formulation of the same active agent.

A preferred liquid dosage composition of the invention exhibits in comparative pharmacokinetic testing with a non-nanoparticulate formulation of the same active agent, administered at the same dosage, a Cₘₐₓ which is at least about 10%, at least about 20%, at least about 30%, at least about 40%, at least about 50%, at least about 60%, at least about 70%, at least about 80%, at least about 90%, or at least about 100% greater than the Cₘₐₓ exhibited by the non-nanoparticulate formulation of the same active agent.

A preferred liquid dosage composition of the invention exhibits in comparative pharmacokinetic testing with a non-nanoparticulate formulation of the same active agent; administered at the same dosage, an AUC which is at least about 10%, at least about 20%, at least about 30%, at least about 40%, at least about 50%, at least about 60%, at least about 70%, at least about 80%, at least about 90%, or at least about 100% greater than the AUC exhibited by the non-nanoparticulate formulation of the same active agent.

Any liquid dosage composition giving the desired pharmacokinetic profile is suitable for administration according to the present methods. Exemplary types of formulations giving such profiles are liquid dispersions, gels, aerosols, ointments, creams, etc.

### H. The Pharmacokinetic Profiles of the Active Agent Compositions of the Invention are not Affected by the Fed or Fasted State of the Subject Ingesting the Compositions

The invention encompasses a liquid dosage form of a nanoparticulate active agent composition wherein the pharmacokinetic profile of the active agent is preferably not substantially affected by the fed or fasted state of a subject ingesting the composition, when administered to a human. This means that there is no substantial difference in the quantity of active agent absorbed or the rate of active agent absorption when the nanoparticulate active agent compositions are administered in the fed versus the fasted state.

The invention also encompasses an active agent composition in which administration of the composition to a subject in a fasted state is bioequivalent to administration of the composition to a subject in a fed state. "Bioequivalency" is preferably established by a 90% Confidence Interval (CI) of between 0.80 and 1.25 for both Cₘₐₓ and AUC under U.S. Food and Drug Administration regulatory guidelines, or a 90% CI for AUC of between 0.80 to 1.25 and a 90% CI for Cₘₐₓ of between 0.70 to 1.43 under the European EMEA regulatory guidelines (Tₘₐₓ is not relevant for bioequivalency determinations under USFDA and EMEA regulatory guidelines).

Benefits of a dosage form which substantially eliminates the effect of food include an increase in subject convenience, thereby increasing subject compliance, as the subject does not need to ensure that they are taking a dose either with or without food. This is significant, as with poor subject compliance an increase in the medical condition for which the drug is being prescribed may be observed.

The difference in absorption of the active agent compositions of the invention, when administered in the fed versus the fasted state, preferably is less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, or less than about 3%.

### I. Bioadhesive Liquid Dosage Compositions of Nanoparticulate Active Agents

Bioadhesive liquid dosage compositions of nanoparticulate active agents according to the present invention comprise at least one cationic surface stabilizer, which are described in more detail below. Bioadhesive formulations of nanoparticulate active agents exhibit exceptional bioadhesion to biological surfaces, such as mucous. The term bioadhesion refers to any attractive interaction between two biological surfaces or between a biological and a synthetic surface. In the case of bioadhesive nanoparticulate active agents, the term bioadhesion is used to describe the adhesion between the nanoparticulate active agents and a biological substrate (*i.e*. gastrointestinal mucin, lung tissue, nasal mucosa, *etc*.). *See e.g.,* U.S. Patent No. 6,428,814 for "Bioadhesive Nanoparticulate Compositions Having Cationic Surface Stabilizers," which is specifically incorporated by reference.

There are basically two mechanisms which may be responsible for this bioadhesion phenomena: mechanical or physical interactions and chemical interactions. The first of these, mechanical or physical mechanisms, involves the physical interlocking or interpenetration between a bioadhesive entity and the receptor tissue, resulting from a good wetting of the bioadhesive surface, swelling of the bioadhesive polymer, penetration of the bioadhesive entity into a crevice of the tissue surface, or interpenetration of bioadhesive composition chains with those of the mucous or other such related tissues. The second possible mechanism of bioadhesion incorporates forces such as ionic attraction, dipolar forces, van der Waals interactions, and hydrogen bonds. It is this form of bioadhesion which is primarily responsible for the bioadhesive properties of the liquid dosage compositions of nanoparticulate active agents of the invention. However, physical and mechanical interactions may also play a secondary role in the bioadhesion of such liquid dosage compositions.

The bioadhesive liquid dosage compositions of nanoparticulate active agents of the invention are useful in any situation in which it is desirable to apply the compositions to a biological surface. The bioadhesive liquid dosage compositions coat the targeted surface in a continuous and uniform film which is invisible to the naked human eye.

A bioadhesive liquid dosage composition of a nanoparticulate active agent slows the transit of the dosage form, and some active agent particles would also most likely adhere to tissue other than the mucous cells and therefore give a prolonged exposure to the active agent, thereby increasing absorption and the bioavailability of the administered dosage.

### II. Compositions

The liquid dosage compositions of the invention comprise at least one nanoparticulate active agent, at least one surface stabilizer adsorbed on or associated with the surface of the active agent, and at least one osmotically active crystal growth inhibitor.

The liquid dosage compositions can additionally comprise one or more non-toxic physiologically acceptable carriers, adjuvants, or vehicles, collectively referred to as carriers. The liquid dosage compositions can be formulated for various routes of administration including but not limited to, oral, rectal, ocular, parenteral injection (*e.g*., intravenous, intramuscular, or subcutaneous), pulmonary, nasal, vaginal, colonic, local (*e.g*., drop form), buccal, intracisternal, intraperitoneal, topical administration, and the like. In addition, the liquid dosage composition may be formulated into any suitable dosage form, such as a liquid dispersion, oral suspension, gel, aerosol, ointment, cream, controlled release formulation, fast melt formulation, lyophilized formulation, tablet, capsule, delayed release formulation, extended release formulation, pulsatile release formulation, and mixed immediate release and controlled release formulation.

### A. Active Agent Particles

In the present invention, one important aspect is that certain surface stabilized nanoparticulate active agents form needle-like crystals in a liquid dosage composition in the absence of an osmotically active crystal growth inhibitor. In preferred embodiments of the invention, the active agent is of a type that forms undesirable crystals during storage and/or heat sterilization, even though the nanoparticulate active agent has at least one surface stabilizer adsorbed or associated with the surface thereof.

Accordingly, it has been surprisingly found that the addition of one or more osmotically active crystal growth inhibitors to a liquid dosage composition of a nanoparticulate active agent results in a liquid dosage composition comprising stable nanoparticulate active agents. This is particularly beneficial for dilute liquid dosage compositions, which can have particular applicability to pediatric administrations.

The nanoparticulate active agent particles present in the liquid dosage compositions of the invention have an effective average particle size of less than about 2 microns and are poorly soluble and dispersible in at least one liquid media. The liquid media is preferably water, but can also be, for example, aqueous salt solutions, safflower oil, or a solvent such as ethanol, t-butanol, hexane, or glycol.

"Poorly soluble active agents" or "poorly soluble drugs" as used herein means those having a solubility in a liquid dispersion media of less than about 30 mg/ml under ambient conditions. In other embodiments of the invention, the active agent preferably has a solubility in the liquid dispersion media of less than about 20 mg/ml, less than about 10 mg/ml, or less than about 1 mg/ml. The pH of aqueous dispersion media can be adjusted by techniques known in the art.

An active agent can be a pharmaceutical or a diagnostic agent such as a contrast agent or any other type of diagnostic material. The therapeutic or diagnostic agent exists as a crystalline phase, a semi-crystalline phase, an amorphous phase, a semi-amorphous phase, or a mixture thereof.

The active agent can be selected from a variety of known classes of drugs, including, for example, proteins, peptides, NSAIDS, COX-2 inhibitors, nutraceuticals, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives (hypnotics and neuroleptics), astringents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immuriological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines.

Examples of representative poorly water soluble active agents useful in this invention include, but are not limited to, acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifene, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, and acetylsalicylate.

Illustrative nutraceuticals include, but are not limited to, dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.

A description of these classes of active agents and a listing of species within each class can be found in Martindale, The Extra Plzannacopoeia, 31st Edition (The Pharmaceutical Press, London, 1996), specifically incorporated herein by reference. The drugs can be commercially available and/or can be prepared by techniques known in the art.

### B. Surface Stabilizers

Surface stabilizers useful herein preferably physically adhere, adsorb, or associate with the surface of the nanoparticulate active agent, but do not chemically react with the active agent or itself. Individual molecules of the surface stabilizer are preferably essentially free of intermolecular crosslinkages.

Useful surface stabilizers which can be employed in the invention include, but are not limited to, known organic and inorganic pharmaceutical compounds. Such compounds include, for example, various polymers, low,molecular weight oligomers, natural products, and surfactants. Preferred surface stabilizers include polymeric, nonionic, anionic, cationic, and zwitterionic surfactants. Representative examples of surface stabilizers are disclosed in U.S. Patent Nos. 6,267,989 and 6,264,922, the contents of which are hereby incorporated by reference.

Representative examples of surface stabilizers include but are not limited to hydroxypropylmethylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g.,* the commercially available Tweens^{®} such as *e.g*., Tween 20^{®} and Tween 80^{®} (ICI Speciality Chemicals)); polyethylene glycols (*e.g*., Carbowaxs 3550^{®} and 934^{®} (Union Carbide)), polyoxyethylene stearates, colloidal silicon dioxide, phosphates, carboxymethylcellulose calcium, carboxymethylcellulose sodium, methyl cellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronics F68^{®} and F108^{®}, which are block copolymers of ethylene oxide and propylene oxide); poloxamines (*e.g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like such as Plasdone® S630 in a 60:40 ratio of the pyrrolidone and acetate.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexadecyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dirnethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂-₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂-₁₄) dimethyl 1-naphthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenaalkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quaternized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric cationic surface stabilizers are any nonpolymeric compound, such as benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ includes at least one heteroatom;
(ix) two of R₁-R₄ are CH₃, one of R₁-R₄ is C₆H₅CH₂, and one of R₁-R₄ includes at least one halogen;
(x) two of R₁-R₄ are CH₃, one of R₁-R₄ is C₆H₅CH₂, and one of R₁-R₄ includes at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quatemium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Many of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

Some surface stabilizers are also referred to as pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference. However, although some of the compounds listed above are also sometimes classified as "excipients" in certain references, when employed as surface stabilizers in the manner described herein, the compounds do not function merely as vehicles and are not considered inert and are thus not "excipients" within the common meaning of the word.

### C. Osmotically Active Crystal Growth Inhibitors

Useful osmotically active crystal growth inhibitors for the liquid dosage composition of the invention: (1) are at least partially soluble in the liquid phase of the composition; and (2) do not appreciably solubilize the nanoparticulate active agent, as solubilization of the active agent destabilizes the composition.

Specifically, osmotically active crystal growth inhibitors according to the invention are compounds that will prevent or inhibit crystal growth of the nanoparticulate active agent. Without limitation, suitable crystal growth inhibitors include: (1) compounds which are liquids at room temperature, such as glycerol and propylene glycol, (2) nonionic compounds which are solids at room temperature, such as mannitol, sucrose, glucose, fructose, mannose, lactose, xylitol, sorbitol, trehalose, or any of the commonly employed mono-, di-, and polysaccharides, sugars, and sugar alcohols, and (3) ionic species such as sodium chloride, potassium chloride, magnesium chloride, or any of the commonly employed salts.

While the inventors do not wish to be bound by theoretical mechanisms, the crystal growth inhibitors are believed to function by decreasing the thermodynamic activity of water and possibly by interfering with micellar solubilization of the active agent when micelle-forming surface inhibitors are present.

### D. Other Pharmaceutical Excipients

Pharmaceutical compositions according to the present invention may also include one or more fillers, suspending agents, sweeteners, flavoring agents, preservatives, buffers, wetting agents, effervescent agents, and other excipients depending upon the route of administration and the dosage form desired. Such excipients are known in the art.

Examples of sweeteners or taste maskants are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable aqueous and nonaqueous carriers, solvents, or vehicles and/or mixtures of any of the foregoing. Examples of diluents include starch, sorbitol, sucrose, and glucose.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

The nanoparticulate compositions may also contain adjuvants such as preserving, emulsifying, and dispensing agents. Prevention of the growth of microorganisms can also be ensured by various antibacterial and antifungal agents, such as parabens, chlorobutanol, phenol, sorbic acid, and the like. It may also be desirable to include isotonic agents, such as sugars, sodium chloride, and the like. Prolonged physiological absorption of the injectable pharmaceutical form can be brought about by the use of agents delaying absorption, such as aluminum monostearate and gelatin.

### E. Nanoparticulate Active Agent Particle Size

The compositions of the invention comprise one or more nanoparticulate active agents which have an effective average particle size of less than about 2000 nm (*i.e*., 2 microns).

In a preferred embodiment of the invention, the active agent nanoparticles have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the active agent particles have a particle size less than the effective average, by weight, *i.e*., less than about 2000 nm, about 1900 nm, about 1800 nm, etc., when measured by the above-noted techniques. In other embodiments of the invention, preferably at least about 70%, at least about 90%, at least about 95%, or at least about 99% of the active agent particles have a particle size of less than the effective average, *i.e.,* less than about 2000 nm, about 1900 nm, about 1800 nm, etc.

In the present invention, the value for D50 of a nanoparticulate active agent composition is the particle size below which 50% of the active agent particles fall, by weight. Similarly, D90 and D95 are the particle size below which 90% and 95%, respectively, of the active agent particles fall, by weight.

### F. Concentration of Nanoparticulate Active Agents, Surface Stabilizers, and Osmotically Active Crystal Growth Inhibitors,

The relative amounts of an active agent used in the present invention and one or more surface stabilizers can vary widely. The optimal amount of the individual components depends, for example, upon one or more of the physical and chemical attributes of the particular active agent selected, such as the hydrophilic lipophilic balance (HLB), melting point, and the surface tension of water solutions of the surface stabilizer, *etc.*

Preferably, the concentration of at least one active agent can vary from about 99.5% to about 0.001%, preferably from about 95% to about 0.1%, and preferably from about 90% to about 0.5%, by weight, based on the total combined dry weight of the active agent and at least one surface stabilizer, not including other excipients. Higher concentrations of the active agent are generally preferred from a dose and cost efficiency standpoint.

Preferably, the concentration of at least one surface stabilizer can vary from about 0.5% to about 99.999%, from about 5.0% to about 99.9%, or from about 10% to about 99.5%, by weight, based on the total combined dry weight of the active agent and at least one surface stabilizer, not including other excipients.

The ratio of the active agent to a secondary surface stabilizer, when present, can preferably vary from about 500:1 to about 5:1, from about 350:1 to about 10:1, or from about 100:1 to about 20:1, by weight.

In a preferred embodiment, the ratio of the active agent to a polymeric surface modifier can vary from about 20:1 to about 1:10, from about 10:1 to about 1:5, or from about 5:1 to about 1:1, by weight.

To reduce, inhibit, or prevent the occurrence of crystal growth of the active agent, especially during storage or heat sterilization, a liquid dispersion containing an active agent is treated with an osmotically active crystal growth inhibitor. The amount of the crystal growth inhibitor can preferably range from about 0.1% to about 95% concentration by weight of the liquid dosage composition, and preferably from about 0.5% to about 90% concentration by weight of the liquid dosage composition.

The amount of osmotically active crystal growth inhibitor will depend upon the particular crystal growth inhibitor utilized and the active agent present in the liquid dosage form, among other factors. Useful amounts of suitable crystal growth inhibitors can be determined using simple screening tests by one of skill in the art.

In one embodiment, liquid dosage compositions of the present invention comprise at least one active agent, at least one surface stabilizer, at least one osmotically active crystal growth inhibitor, and in some embodiments of the invention, water. If a crystal growth inhibitor that is a solid at room temperature (such as mannitol or sodium chloride) is used as the crystal growth inhibitor, the upper concentration limit of the crystal growth inhibitor is controlled by the solubility of the inhibitor in the liquid phase of the dosage form.

In some embodiments, the invention preferably encompasses liquid dosage compositions comprising one or more taste maskants, flavorants, colorants, antimicrobial preservatives, sweeteners, viscosity modifiers, antioxidants, and/or other excipients.

### III. Methods of Making Nanoparticulate Formulations

The liquid dosage compositions of nanoparticulate active agents can be made using, for example, milling, homogenization, or precipitation techniques. The osmotically active crystal growth inhibitor is contacted with the nanoparticulate active agent either before, during, or after active agent particle size reduction.

Exemplary methods of making nanoparticulate active agent compositions are described in the '684 patent. Methods of making nanoparticulate active agent compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

The resultant nanoparticulate active agent compositions or dispersions can be utilized in liquid dosage formulations, such as liquid dispersions, aerosols, controlled release formulations, capsules, delayed release formulations, extended release formulations, pulsatile release formulations, mixed immediate release and controlled release formulations, etc.

### A. Milling to Obtain Nanoparticulate Active Agent Dispersions

Milling an active agent to obtain a nanoparticulate active agent dispersion comprises dispersing active agent particles in a liquid dispersion media in which the active agent is poorly soluble, followed by applying mechanical means in the presence of grinding media to reduce the particle size of the active agent to the desired effective average particle size. The dispersion media can be, for example, water, safflower oil, ethanol, t-butanol, glycerin, polyethylene glycol (PEG), hexane, or glycol.

The active agent particles can be reduced in size preferably in the presence of at least one surface stabilizer and optionally at least one osmotically active crystal growth inhibitor. The dispersion formed by the milling techniques can then be diluted using an additional amount of crystal growth inhibitor, as described herein. Alternatively, the active agent particles can be contacted with one or more surface stabilizers and/or at least one osmotically active crystal growth inhibitor after attrition. Other compounds, such as a diluent, can be added to the active agent composition during the size reduction process. Dispersions can be manufactured continuously or in a batch mode.

### B. Precipitation to Obtain Nanoparticulate Active Agents

Another method of forming the desired liquid dosage composition of a nanoparticulate active agent is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving at least one active agent in a suitable solvent; (2) adding the formulation from step (1) to a solution comprising at least one surface stabilizer and preferably at least one osmotically active crystal growth inhibitor; and (3) precipitating the formulation from step (2) using an appropriate non-solvent. The method can be followed by removal of any formed salt, if present, by dialysis or diafiltration and concentration of the dispersion by conventional means.

### C. Homogenization to Obtain Nanoparticulate Active Agents

Exemplary homogenization methods of preparing nanoparticulate active agent compositions are described in U.S. Patent No. 5,510,118, for "Process of Preparing Therapeutic Compositions Containing Nanoparticles." Such a method comprises dispersing active agent particles in a liquid dispersion media in which the active agent is poorly soluble, followed by subjecting the dispersion to homogenization to reduce the particle size of the active agent to the desired effective average particle size. The active agent particles can be reduced in size in the presence of at least one surface stabilizer and/or at least one osmotically active crystal growth inhibitor. Alternatively, the active agent particles can be contacted with one or more surface stabilizers and/or at least one osmotically active crystal growth inhibitor either before or after attrition. Other compounds, such as a diluent, can be added to the active agent composition either before, during, or after the size reduction process. Dispersions can be manufactured continuously or in a batch mode.

### IV. Methods of Using the Liquid Dosage Compositions of the Invention

The liquid dosage compositions of the invention can be administered to a subject via any conventional liquid dosage method including, but not limited to, orally, rectally, ocularly, parenterally (*e.g*., intravenous, intramuscular, or subcutaneous), intracisternally, pulmonary, intravaginally, intraperitoneally, locally (*e.g*., ointments or drops), or as a buccal or nasal spray. As used herein, the term "subject" is used to mean an animal, preferably a mammal, including a human or non-human. The terms "patient" and "subject" may be used interchangeably.

The liquid dosage compositions of the invention can be used to treat any condition for which the active agent present in the composition is useful. The liquid dosage compositions of the invention are particularly useful in treating patient populations such as pediatrics and the elderly. Exemplary conditions which can be treated with liquid dosage compositions include, but are not limited to, neoplastic diseases, breast cancer, endometrial cancer, uterine cancer, cervical cancer, prostate cancer, renal cancer, hormone replacement therapy in post-menopausal women, endometriosis, hirsutism, dysmenorrhea, uterine bleeding, HIV wasting, cancer wasting, migraine headache, cachexia, anorexia, castration, oral contraception, motion sickness, emesis related to cytotoxic drugs, gastritis, ulcers, dyspepsia, gastroenteritis, including collitis and food poisoning, inflammatory bowel disease, Crohn's disease, migraine headaches, and any other condition which is accompanied by the symptoms of nausea and vomiting. Other conditions which can be treated with the liquid dosage compositions of the invention include, but are not limited to, pain, inflammation, arthritis, cancer, kidney disease, osteoporosis, Alzheimer's disease, and familial adenomatous polyposis. Yet other conditions which can be treated with the liquid dosage compositions of the invention include, but are not limited to, osteoarthritis, rheumatoid arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma.

Liquid dosage compositions suitable for parenteral injection may include physiologically acceptable sterile aqueous or nonaqueous solutions, dispersions, suspensions or emulsions, and sterile powders for reconstitution into sterile injectable solutions or dispersions. Proper fluidity can be maintained, for example, by the use of a coating such as lecithin, by the maintenance of the required particle size in the case of dispersions, and by the use of surfactants.

Liquid dosage compositions, preferably for oral administration, include pharmaceutically acceptable emulsions, solutions, suspensions, syrups, and elixirs. In addition to the active agent, surface stabilizer and osmotically active crystal growth inhibitor, the liquid dosage compositions may include inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propylene glycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like. Further, if sufficient amounts of the osmotically active crystal growth inhibitor are used in the liquid dosage composition, the crystal growth inhibitor can also function as a diluent.

The final volume of the liquid dosage composition depends upon the dose of active agent needed such that the volume to be administered to a patient is measurable and useful. In some embodiments the diluent can be the same material as the osmotically active crystal growth inhibitor, such as an appropriate polyol (*e.g*., glycerol).

The effective amounts of the active agent of the composition of the invention can be determined empirically and can be employed in pure form or, where such forms exist, in pharmaceutically acceptable salt, ester, or prodrug form. Actual dosage levels of the active agent in the liquid dosage compositions of the invention may be varied to obtain an amount of the active agent that is effective to obtain a desired therapeutic response for a particular composition and method of administration and the condition to be treated. The selected dosage level therefore depends upon the desired therapeutic effect, the route of administration, the potency of the administered active agent, the desired duration of treatment, and other factors.

Dosage unit compositions may contain such amounts of such submultiples thereof as may be used to make up the daily dose. It will be understood, however, that the specific dose level for any particular subject will depend upon a variety of factors: the type and degree of the cellular or physiological response to be achieved; activity of the specific agent or composition employed; the specific agent(s) or composition employed; the age, body weight, general health, sex, and diet of the patient; the time of administration, route of administration, and rate of excretion of the active agent; the duration of the treatment; active agents used in combination or coincidental with the specific active agent; and like factors well known in the medical arts.

In some embodiments, the dispersion obtained directly after milling is too concentrated and difficult to measure to provide a consistent dosage unit, and therefore the dispersion is typically diluted. Additional taste masking agents can also be used depending on the particular ingredients of the dosage unit.

The following examples are given to illustrate the present invention. It should be understood, however, that the invention is not to be limited to the specific conditions or details described in these examples. Throughout the specification, any and all references to a publicly available document, including a U.S. patent, are specifically incorporated by reference.

### Examples

The purpose of Examples 1-3 was to prepare dilute nanoparticulate active agent dispersions of Compound A, a compound having anti-inflammatory and analgesic properties and which also forms needle-like crystals upon storage in the absence of an appropriate osmotically active crystal growth inhibitor. In addition, the examples test the prepared compositions for stability in the presence and absence of an osmotically active crystal growth inhibitor.

The stability of nanoparticulate Compound A formulations was determined by visual inspection of the mixtures to verify whether or not needle-like crystals formed.

### Example 1

An aqueous nanoparticulate colloidal dispersion (NCD) comprising 32.5% (w/w) Compound A, 6.5% (w/w) copovidone (Plasdone® S-630; International Specialty Products, Wayne, NJ), and 0.464% (w/w) dioctyl sodium sulfosuccinate (DOSS; Cytec Industries) was prepared by milling for 3.8 hours under high energy milling conditions in a Netzsch LMZ-10 horizontal media mill (Netzsch Inc., Exton, PA) with 500 µm polymeric attrition media.

The final mean particle size (by weight) of the Compound A particles was 161 nm, with a D50 < 145 nm, D90 < 263 nm, and D95 < 307 nm, as measured using a Horiba LA-910 Laser Scattering Particle Size Distribution Analyzer (Horiba Instruments, Irvine, CA).

The concentrated NCD was then diluted with preserved water and glycerol (the osmotically active crystal growth inhibitor) to between 0.5% and 3.0% Compound A (w/w) (Samples 1A-1F), as shown in the Table 1, below. Samples 1A and 1C were free of glycerol. The preserved water consisted of an aqueous solution of the sodium salts of methyl and propyl parabens (0.206% and 0.022% respectively) and 0.1% citric acid.

The compositions were evaluated for physical stability by optical microscopy after storage for 3 days at 40°C.

| **Table 1** | | | | | |
|---|---|---|---|---|---|
| **Sample Ref.** | **Cmpd. A** **(w/w)** | **S-630** **(w/w)** | **DOSS** **(w/w)** | **Glycerol** **(w/w)** | **Stability Data at 40°C after 3 days (microscope)** |
| Sample 1A | 0.50% | 0.10% | 0.01% | 0.00% | needles present |
| Sample 1B | 0.50% | 0.10% | 0.01% | 25.00% | needles present |
| Sample 1C | 2.80% | 0.60% | 0.04% | 0.00% | needles present |
| Sample 1D | 2.80% | 0.60% | 0.04% | 24.00% | needles present |
| Sample 1E | 0.50% | 0.10% | 0.01% | 74.00% | no needles visible |
| Sample 1F | 2.80% | 0.60% | 0.04% | 71.00% | no needles visible |

The results of this experiment unexpectedly show that dilutions which contained >70% by weight glycerol (Samples 1E and 1F) were stable, *i.e.,* no crystal needles of Compound A were visible upon visual inspection of the mixture under a microscope, following a three day storage period.

### Example 2

The nanoparticulate colloidal dispersion (NCD) of Compound A, with Plasdone® S-630 and DOSS as surface stabilizers as described in Example 1, was diluted with glycerol and preserved water and examined for stability at different weight percentages of the final product. Compound A, glycerol, Plasdone® S-630, and DOSS had a final weight percentage as shown in Table 2.

The compositions were evaluated for physical stability by optical microscopy after storage for 34 days at 40°C.

| **Table 2** | | | | | |
|---|---|---|---|---|---|
| **Sample Ref.** | **Cmpd. A** **(w/w)** | **S-630** **(w/w)** | **DOSS** **(w/w)** | **Glycerol** **(w/w)** | **Stability Data at 40°C after 34 days (microscope)** |
| Sample 2A | 3.00% | 0.60% | 0.04% | 75.00% | no needles visible |
| Sample 2B | 0.50% | 0.10% | 0.01% | 75.00% | no needles visible |
| Sample 2C | 0.50% | 0.10% | 0.01% | 90.00% | no needles visible |

The results of this experiment show that final dilutions containing (1) between 0.5% (w/w) and 3% (w/w) of Compound A and (2) at least 75% by weight glycerol were stable, *i.e.,* the nanoparticles of Compound A did not form needle-like crystals.

### Example 3

A nanoparticulate colloidal dispersion (NCD) comprising 15% Compound A, 3% Plasdone® S-630, and 0.214% DOSS in preserved water (all (w/w) basis; 400 g total batch size) was prepared by milling for 170 min. under high energy milling conditions in a DYNO®-Mill KDL (Willy A. Bachofen AG, Maschinenfabrik, Basel, Switzerland) equipped with a 300 cc recirculation chamber and utilizing 500 µm polymeric attrition media.

The final (weight) mean particle size of the Compound A particles was 108 nm, with D50 < 107 nm, D90 < 170 nm, and D95 < 198 nm, as measured using a Horiba LA-910 Laser Scattering Particle Size Distribution Analyzer (Horiba Instruments, Irvine, CA).

The preserved water consisted of an aqueous solution of the sodium salts of methyl and propyl parabens (0.206% and 0.022% respectively) and 0.1% citric acid. The concentrated NCD was then diluted with glycerol and preserved water to 3% (w/w) Compound A, as shown in the Table 3 below.

The compositions were evaluated for physical stability by optical microscopy after storage for 10 days at 40°C.

| **Table 3** | | | | | |
|---|---|---|---|---|---|
| **Sample Ref.** | **Cmpd. A** **(w/w)** | **S-630** **(w/w)** | **DOSS** **(w/w)** | **Glycerol** **(w/w)** | **Stability Data at 40°C after 10 days (microscope)** |
| Sample 3A | 3.00% | 0.60% | 0.04% | 40.00% | needles present |
| Sample 3B | 3.00% | 0.60% | 0.04% | 50.00% | needles present |
| Sample 3C | 3.00% | 0.60% | 0.04% | 60.00% | a few needles |
| Sample 3D | 3.00% | 0.60% | 0.04% | 70.00% | no needles visible |
| Sample 3E | 3.00% | 0.60% | 0.04% | 80.00% | no needles visible |

The results of this experiment show that dilutions containing 3% (w/w) of Compound A develop stability as the amount of glycerol increases. As shown in Table 3, as the amount of glycerol in the final mixture approaches 60% to 70% (w/w), the number of crystals is reduced to a few or zero, and the stability of the final mixture becomes apparent. These results are consistent with the results of Example 2.

The data also show that glycerol overcomes crystal growth and a liquid dosage composition of the invention can be stable over time, even at an elevated temperature.

The purpose of Examples 4 and 5 was to prepare nanoparticulate dispersions of Compound B, a compound having anti-inflammatory and analgesic properties and which also undergoes crystal growth upon storage in the absence of an appropriate osmotically active crystal growth inhibitor, and to test the prepared compositions for stability in the presence and absence of an osmotically active crystal growth inhibitor. Stability was determined by static light scattering methods to verify whether or not larger crystals of the Compound B formed. The crystal growth inhibitor used in Example 5 was mannitol.

### Example 4

A nanoparticulate colloidal dispersion (NCD) of Compound B having 5% (w/w) Compound B, 1% (w/w) Kollidon® K17 PF (PVP), and 0.05% (w/w) sodium deoxycholate (NaDOC; Spectrum Quality Products Inc., New Brunswick, NJ) as a secondary surface stabilizer was milled for 2 hours under high energy milling conditions in a DYNO®-Mill KDL (Willy A. Bachofen AG, Maschinenfabrik, Basel, Switzerland) equipped with a 150 cc batch chamber and utilizing 200 µm polymeric attrition media.

The final (weight) mean particle size of the Compound B particles was 87 nm, with D50 < 83 nm, D90 < 122 nm, and D95 < 145 nm, as measured using a Horiba LA-910 Laser Scattering Particle Size Distribution Analyzer (Horiba Instruments, Irvine, CA).

Compound B, PVP, and NaDOC had final weight percentages as shown in Table 4 (all measurements in Table 4 are w/w). As further shown in Table 4, Sample 4 was free of mannitol. Dₘₑₐₙ is the initial mean (weight) particle size, and Dₘₑₐₙ after storage is the mean (weight) particle size of the Compound B composition measured after storage at room temperature for 24 hours.

| **Table 4** | | | | | | |
|---|---|---|---|---|---|---|
| **Sample Ref.** | **Compound B** | **PVP** | **NaDOC** | **Mannitol** | **Initial Dₘₑₐₙ** | **Dₘₑₐₙ after storage** |
| Sample 4 | 5.0% | 1.0% | 0.05% | 0.00% | 87 nm | 856 nm |

The data shows that in the absence of an osmotically stable crystal growth inhibitor, a dispersion of Compound B exhibits dramatic particle size growth - from 87 nm to 856 nm, with D50 < 770 nm, D90 < 1783 nm, and D95 < 2084 nm, -- even after storage for only 24 hours at room temperature. Thus, the dispersion of Compound B is highly unstable.

### Example 5

A nanoparticulate colloidal dispersion (NCD) of Compound B having 5% (w/w) Compound B, 1% (w/w) Kollidon® K17 PF (PVP), and 0.05% (w/w) sodium deoxycholate (NaDOC; Spectrum Quality Products Inc., New Brunswick, NJ) as a secondary surface stabilizer was milled for 5.5 hours under high energy milling conditions in a DYNO®-Mill KDL (Willy A. Bachofen AG, Maschinenfabrik, Basel, Switzerland) equipped with a 600 cc batch chamber and utilizing 200 µm polymeric attrition media.

The final (weight) mean particle size of the Compound B particles was 87 nm, with D90 < 130 nm, as measured using a Horiba LA-910 Laser Scattering Particle Size Distribution Analyzer (Horiba Instruments, Irvine, CA).

Portions of mannitol were added to the Compound B NCD to yield different weight percentages of mannitol. The samples were then examined for stability upon storage at room temperature for 4 days. At the time of mannitol addition, the Compound B particle size was ca. 105 nm. The Compound B, PVP, NaDOC, and mannitol had final weight percentages as shown in Table 5 (all measurements in Table 5 are w/w).

In Table 5, Dₘₑₐₙ is the initial mean (weight) particle size, and Dₘₑₐₙ after storage is the mean (weight) particle size of the Compound B composition measured after storage at room temperature for 4 days.

| **Table 5** | | | | | | |
|---|---|---|---|---|---|---|
| **Sample Ref.** | **Compound B** | **PVP** | **NaDOC** | **Mannitol** | **Initial Dₘₑₐₙ** | **Dₘₑₐₙ after storage** |
| Sample 5A | 4.8% | 0.95% | 0.048% | 5.00% | 104 nm | 205 nm |
| Sample 5B | 4.5% | 0.90% | 0.045% | 10.00% | 105 nm | 158 nm |

Sample 5A had an initial mean (weight) particle size of 104 nm, with D50 < 101 nm, D90 < 139 nm, and D95 < 150 nm. After storage at room temperature for 4 days, Sample 5A had a mean (weight) particle size of 205 nm, with D50 < 114 nm, D90 < 268 nm, and D95 < 691 nm, as measured using a Horiba LA-910 Laser Scattering Particle Size Distribution Analyzer (Horiba Instruments, Irvine, CA).

Sample 5B had an initial mean (weight) particle size of 105 nm, with D50 < 100 nm, D90 < 144 nm, and D95 < 160 nm. After storage at room temperature for 4 days, Sample 5B had a mean (weight) particle size of 158 nm, with D50 < 112 nm, D90 < 227 nm, and D95 < 322 nm, as measured using a Horiba LA-910 Laser Scattering Particle Size Distribution Analyzer (Horiba Instruments, Irvine, CA).

The results of Examples 4 and 5 show that mixtures of nanoparticulate Compound B which contained mannitol (Samples 5A and 5B) were more stable than those without mannitol (Sample 4), *i.e*., dramatically less particle growth was observed after storage at room temperature for the composition comprising mannitol. Moreover, only a small quantity of mannitol is required, by weight, to stabilize the composition against particle size growth.

### Example 6

The purpose of Example 6 was to prepare dispersions of nanoparticulate ketoprofen, a compound having anti-inflammatory and analgesic properties and which also undergoes crystal growth upon storage in the absence of an appropriate osmotically active crystal growth inhibitor, and to test the prepared compositions for stability in the presence and absence of a crystal growth inhibitor. Stability was determined by static light scattering methods to verify whether or not larger crystals of ketoprofen formed. The crystal growth inhibitor used in this example was glycerol.

A nanoparticulate colloidal dispersion having 25% (w/w) ketoprofen, 5% (w/w) hydroxypropylmethylcellulose (HPMC; Pharmacoat® 603, Shin-Etsu), and 0.25% (w/w) dioctyl sodium sulfosuccinate (DOSS) was milled for 13.5 hours under high energy milling conditions in a Netzsch LMZ-2 horizontal media mill with 500 µm polymeric attrition media.

The final (weight) mean particle size of the ketoprofen particles was 167 nm, with D50 < 162 nm, D90 < 226 nm, and D95 < 250 nm, as measured using a Horiba LA-910 Laser Scattering Particle Size Distribution Analyzer (Horiba Instruments, Irvine, CA).

Portions of the ketoprofen NCD were diluted and combined with different weight percentages of glycerol and examined for stability after storage at 40°C for 1 week. The ketoprofen, HPMC, DOSS, and glycerol had final weight percentages as shown in Table 6. As further shown in Table 6, Sample 6A was free of glycerol (all measurements in Table 6 are w/w).

In Table 6, Dₘₑₐₙ is the initial mean (weight) particle size, and Dₘₑₐₙ after storage is the mean (weight) particle size of the Compound B composition measured after storage at 40°C for 1 week.

Sample 6A had an initial mean (weight) particle size of 201 nm, with D50 < 195 nm, D90 < 262 nm, and D95 < 288 nm. After storage at 40°C for 1 week, Sample 6A had a mean (weight) particle size of 231 nm, with D50 < 224 nm, D90 < 294 nm, and D95 < 323 nm, as measured using a Horiba LA-910 Laser Scattering Particle Size Distribution Analyzer (Horiba Instruments, Irvine, CA).

Sample 6B had an initial mean particle size of 186 nm, with 50% < 181 nm, 90% < 248 nm, and 95% < 271 nm. After storage at 40°C for 1 week, Sample 6B had a mean (weight) particle size of 188 nm, with D50 < 184 nm, D90 < 247 nm, and D95 < 267 nm, as measured using a Horiba LA-910 Laser Scattering Particle Size Distribution Analyzer (Horiba Instruments, Irvine, CA).

| **Table 6** | | | | | | |
|---|---|---|---|---|---|---|
| **Sample Ref.** | **Ketoprofen** | **HPMC** | **DOSS** | **Glycerol** | **Initial Dₘₑₐₙ** | **Dₘₑₐₙ after storage** |
| Sample 6A | 2.0% | 1.0% | 0.05% | 0.00% | 201 nm | 231 nm |
| Sample 6B | 2.0% | 1.0% | 0.05% | 25.00% | 186 nm | 188 nm |

The results of this experiment shows that the mixture of nanoparticulate ketoprofen which contained glycerol (Sample 6B) was more stable, *i.e*., much less particle growth was observed after storage at 40°C.

The purpose of Examples 7 and 8 was to prepare dispersions of nanoparticulate triamcinolone acetonide, a glucocorticoid compound having anti-inflammatory properties and which also undergoes crystal growth upon storage in the absence of an appropriate crystal growth inhibitor, and to test the prepared compositions for stability in the presence and absence of a crystal growth inhibitor. Stability was determined by static light scattering methods to verify whether or not larger crystals of triamcinolone acetonide formed. The osmotically active crystal growth inhibitor used in Example 8 was sodium chloride.

### Example 7

A nanoparticulate colloidal dispersion (NCD) of triamcinolone acetonide having 5% (w/w) triamcinolone acetonide and 0.5% (w/w) tyloxapol was milled for 1 hour under high energy milling conditions in a DYNO®-Mill KDL (Willy A. Bachofen AG, Maschinenfabrik, Basel, Switzerland) equipped with a 150 cc batch milling chamber and using 500 µm polymeric attrition media.

The final (weight) mean particle size of the triamcinolone acetonide particles was 182 nm, with D50 < 173 nm, D90 < 262 nm, and D95 < 296 nm, as measured using a Horiba LA-910 Laser Scattering Particle Size Distribution Analyzer (Horiba Instruments, Irvine, CA) and a 0.01% w/w solution of benzalkonium chloride as the dispersing medium.

In the absence of added crystal growth inhibitor, the average particle size of the triamcinolone acetonide dispersion increased by 54 nm to 236 nm, with D50 < 225 nm, D90 < 325 nm, and D95 < 364 nm, after storage at room temperature for 24 hours, as shown in Table 7. Particle size was measured using a Horiba LA-910 Laser Scattering Particle Size Distribution Analyzer (Horiba Instruments, Irvine, CA).

| **Table 7** | | | | | |
|---|---|---|---|---|---|
| **Sample Ref.** | **Triamcinolone Acetonide** | **tyloxapol** | **NaCl** | **Initial Dₘₑₐₙ** | **Dₘₑₐₙ after storage** |
| Sample 7 | 5.0% | 0.5% | 0% | 182 nm | 236 nm |

### Example 8

A nanoparticulate colloidal dispersion (NCD) of triamcinolone acetonide having 5% (w/w) triamcinolone acetonide, 0.5% (w/w) tyloxapol, and 0.5% (w/w) sodium chloride crystal growth inhibitor was milled for 2 hours under high energy milling conditions in a DYNO®-Mill KDL (Willy A. Bachofen AG, Maschinenfabrik, Basel, Switzerland) equipped with a 150 cc batch milling chamber and using 500 µm polymeric attrition media.

The final (weight) mean particle size of the triamcinolone acetonide particles was 149 nm, with D90 < 212 nm, as measured using a Horiba LA-910 Laser Scattering Particle Size Distribution Analyzer (Horiba Instruments, Irvine, CA) and ) using a 0.5% w/w solution of sodium chloride as the dispersing medium.

In the presence of 0.5% w/w sodium chloride crystal growth inhibitor, the average particle size of the triamcinolone acetonide dispersion increased by only 16 nm to 165 nm (D90 < 243 nm) after storage at room temperature for 24 h as shown in Table 8.

| **Table 8** | | | | | |
|---|---|---|---|---|---|
| **Sample Ref.** | **Triamcinolone Acetonide** | **tyloxapol** | **NaCl** | **Initial Dₘₑₐₙ** | **Dₘₑₐₙ after storage** |
| Sample 8 | 5% | 0.5% | 0.5% | 149 nm | 165 nm |

The results of experiments 7 and 8 show that the dispersion of nanoparticulate triamcinolone acetonide which contained sodium chloride (Sample 8) was dramatically more stable than the dispersion lacking sodium chloride (Sample 7), *i.e*., much less particle growth was observed after storage at room temperature for 24 hours.

It will be apparent to those skilled in the art that various modifications and variations can be made in the methods and compositions of the present invention without departing from the spirit and scope of the invention. Thus, it is intended that the present invention cover the modifications and variations provided they come within the scope of the appended claims and their equivalents.

The following pages 47 to 64 contain preferred embodiments. Accordingly, the term "claim" as used therein refers to such a "preferred embodiment".
1. A stable nanoparticulate liquid dosage composition comprising:
   (a) particles of at least one active agent having an effective average particle size of less than about 2000 nm;
   (b) at least one surface stabilizer; and
   (c) at least one osmotically active crystal growth inhibitor.
2. The composition of claim 1, wherein the active agent particles form crystals upon storage or heating in the absence of the crystal growth inhibitor.
3. The composition of claim 1 or claim 2, wherein the osmotically active crystal growth inhibitor is at least partially water-soluble and does not solubilize the nanoparticulate active agent.
4. The composition of any one of claims 1-3, wherein the osmotically active crystal growth inhibitor is selected from the group consisting of glycerol, propylene glycol, mannitol, sucrose, glucose, fructose, mannose, lactose, xylitol, sorbitol, trehalose, a polysaccharide, a mono-polysaccharide, a di-polysaccharides, a sugars, a sugar alcohol, sodium chloride, potassium chloride, magnesium chloride, and an ionic salt.
5. The composition of any one of claims 1-4, wherein the crystal growth inhibitor is selected from the group consisting of glycerol, mannitol, and sodium chloride.
6. The composition of any one of claims 1-5, wherein the amount of the crystal growth inhibitor present in the liquid dosage composition ranges from about 0.1% to about 95% concentration, by weight, or from about 0.5% to about 90% concentration, by weight.
7. The composition of any one of claims 1-6, wherein the effective average particle size of the nanoparticulate active agent particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
8. The composition of any one of claims 1-7, wherein at least about 70%, about 90%, or about 95% of the active agent particles have a particle size less than the effective average particle size.
9. The composition of any one of claims 1-8, wherein the amount of the active agent per ml is equal to or greater than the amount of the active agent per ml of a standard conventional non-nanoparticulate liquid dosage composition of the same active agent.
10. The composition of any one of claims 1-9, wherein the liquid media of the liquid dosage composition is selected from the group consisting of water, safflower oil, ethanol, t-butanol, glycerin, polyethylene glycol (PEG), hexane, and glycol.
11. The composition of any one of claims 1-10, wherein the composition is formulated for administration selected from the group consisting of oral, pulmonary, rectal, ophthalmic, colonic, parenteral, intracisternal, intravaginal, intraperitoneal, local, buccal, nasal, and topical administration.
12. The composition of any one of claims 1-11 formulated into a dosage form selected from the group consisting of liquid dispersions, oral suspensions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.
13. The composition of any one of claims 1-12, wherein the at least one active agent is present in an amount selected from the group consisting of from about 99.5% to about 0.001%, from about 95% to about 0.1%, and from about 90% to about 0.5%, by weight, based on the total combined dry weight of the active agent and at least one surface stabilizer, not including other excipients.
14. The composition of any one of claims 1-13, wherein the at least one surface stabilizer is present in an amount selected from the group consisting of from about 0.5% to about 99.999% by weight, from about 5.0% to about 99.9% by weight, and from about 10% to about 99.5% by weight, based on the total combined dry weight of the active agent and at least one surface stabilizer, not including other excipients.
15. The composition of any one of claims 1-14, wherein the ratio of active agent to a polymeric surface modifier is selected from the group consisting of from about 20:1 to about 1:10, from about 10:1 to about 1:5, and from about 5:1 to about 1:1, by weight.
16. The composition of any one of claims 1-15, comprising at least two surface stabilizers.
17. The composition of claim 16, wherein the ratio of active agent to the second surface stabilizer is selected from the group consisting of from about 500:1 to about 5:1, from about 350:1 to about 10:1, and from about 100:1 to about 20:1, by weight.
18. The composition of any one of claims 1-17, wherein the composition further comprises one or more pharmaceutically acceptable excipients, carriers, or a combination thereof.
19. The composition of any one of claims 1-18, wherein the surface stabilizer is selected from the group consisting of an anionic surface stabilizer, a cationic surface stabilizer, a polymeric surface stabilizer, a nonionic surface stabilizer, and a zwitterionic surface stabilizer.
20. The composition of claim 19, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, and random copolymers of vinyl acetate and vinyl pyrrolidone.
21. The composition of claim 19, wherein the at least one cationic surface stabilizer is selected from the group consisting of a polymer, a biopolymer, a polysaccharide, a cellulosic, an alginate, a nonpolymeric compound, a phospholipid, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MERAPOL™, ALKAQUAT™, alkyl pyridinium salts; amines, amine salts, amine oxides, imide azolinium salts, protonated quaternary acrylamides, methylated quaternary polymers, and cationic guar.
22. The composition of any one of claims 19 to 21, wherein the composition is bioadhesive.
23. The composition of any one of claims 1-22, wherein the active agent is selected from the group consisting of a crystalline phase, an amorphous phase, a semi-crystalline phase, a semi-amorphous phase, and mixtures thereof.
24. The composition of any one of claims 1-23, wherein the one or more active agents have a solubility in water selected from the group consisting of less than about 30 mg/ml, less than about 20 mg/ml, less than about 10 mg/mI, and less than about 1 mg/ml, under ambient conditions.
25. The composition of any one of claims 1-24, wherein the active agent comprises anti-inflammatory and analgesic properties.
26. The composition of any one of claims 1-25, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.
27. The composition of any one of claims 1-26, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.
28. The composition of any one of claims 1-26, wherein the active agent is selected from the group consisting of acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifene, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, and acetylsalicylate.
29. The composition of any one of claims 1-28, wherein the viscosity of the composition, at a shear rate of 0.1 (1/s), is selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mFa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·sto about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s.
30. The composition of any one of claims 1-29, wherein the viscosity of the composition is selected from the group consisting of less than about 1/200, less than about 1/100, less than about 1/50, less than about 1/25, and less than about 1/10 of the viscosity of a standard conventional non-nanoparticulate liquid dosage composition of the same active agent at about the same concentration per ml of active agent.
31. The composition of any one of claims 1-30, wherein the viscosity of the composition is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional non-nanoparticulate liquid dosage composition of the same active agent at about the same concentration per ml of active agent.
32. The composition of any one of claims 1-31, wherein the Tₘₐₓ of the active agent, when assayed in the plasma of a mammalian subject following administration, is less than the Tₘₐₓ for a conventional, non-nanoparticulate form of the same active agent, administered at the same dosage.
33. The composition of claim 32, wherein the Tₘₐₓ is selected from the group consisting of not greater than about 90%, not greater than about 80%, not greater than about 70%, not greater than about 60%, not greater than about 50%, not greater than about 30%, not greater than about 25%, not greater than about 20%, not greater than about 15%, and not greater than about 10% of the Tₘₐₓ, exhibited by a non-nanoparticulate formulation of the same active agent, administered at the same dosage.
34. The composition of any one of claims 1-33, wherein the Cₘₐₓ of the active agent, when assayed in the plasma of a mammalian subject following administration, is greater than the Cₘₐₓ for a conventional, non-nanoparticulate form of the same active agent, administered at the same dosage.
35. The composition of claim 34, wherein the Cₘₐₓ is selected from the group consisting of at least about 10%, at least about 20%, at least about 30%, at least about 40%, at least about 50%, at least about 60%, at least about 70%, at least about 80%, at least about 90%, and at least about 100% greater than the Cₘₐₓ exhibited by a non-nanoparticulate formulation of the same active agent, administered at the same dosage.
36. The composition of any one of claims 1-35, wherein the AUC of the active agent, when assayed in the plasma of a mammalian subject following administration, is greater than the AUC for a conventional, non-nanoparticulate form of the same active agent, administered at the same dosage.
37. The composition of claim 36, wherein the AUC is selected from the group consisting of at least about 10%, at least about 20%, at least about 30%, at least about 40%, at least about 50%, at least about 60%, at least about 70%, at least about 80%, at least about 90%, and at least about 100% greater than the AUC exhibited by a non-nanoparticulate formulation of the same active agent, administered at the same dosage.
38. The composition of any one of claims 1-37 which does not produce significantly different absorption levels when administered under fed as compared to fasting conditions.
39. The composition of claim 38, wherein the difference in absorption of the active agent composition of the invention, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%.
40. The composition of any one of claims 1-39, wherein administration of the composition to a subject in a fasted state is bioequivalent to administration of the composition to a subject in a fed state, when administered to a human.
41. The composition of claim 40, wherein "bioequivalency" is established by a 90% Confidence Interval of between 0.80 and 1.25 for both Cₘₐₓ and AUC, when administered to a human.
42. The composition of claim 40, wherein "bioequivalency" is established by a 90% Confidence Interval of between 0.80 and 1.25 for AUC and a 90% Confidence Interval of between 0.70 to 1.43 for Cₘₐₓ, when administered to a human.
43. A method of making a liquid dosage composition of a stable nanoparticulate active agent comprising contacting particles of at least one active agent with at least one surface stabilizer for a time and under conditions sufficient to provide a nanoparticulate active agent composition wherein:
   (a) the active agent particles have an effective average particle size of less than about 2 microns; and
   (b) at least one osmotically active crystal growth inhibitor is added to the nanoparticulate active agent composition either before, during, or after the active agent particle size reduction.
44. The method of claim 43, wherein said contacting comprising grinding.
45. The method of claim 44, wherein said grinding comprising wet grinding.
46. The method of claim 43, wherein said contacting comprises homogenizing.
47. The method of claim 43, wherein said contacting comprises:
   (a) dissolving the particles of at least one active agent in a solvent;
   (b) adding the resulting solution of the active agent to a solution comprising at least one surface stabilizer; and
   (c) precipitating the solubilized active agent and at least one surface stabilizer by the addition thereto of a non-solvent.
48. The method of any one of claims 43-47, wherein the active agent particles form crystals upon storage or heating in the absence of the crystal growth inhibitor.
49. The method of any one of claims 43-48, wherein the osmotically active crystal growth inhibitor is at least partially water-soluble and does not solubilize the nanoparticulate active agent.
50. The method of any one of claims 43-49, wherein the osmotically active crystal growth inhibitor is selected from the group consisting of glycerol, propylene glycol, mannitol, sucrose, glucose, fructose, mannose, lactose, xylitol, sorbitol, trehalose, a polysaccharide, a mono-polysaccharide, a di-polysaccharides, a sugars, a sugar alcohol, sodium chloride, potassium chloride, magnesium chloride, and an ionic salt.
51. The method of any one of claims 43-50, wherein the crystal growth inhibitor is selected from the group consisting of glycerol, mannitol, and sodium chloride.
52. The method of any one of claims 43-51, wherein the amount of the crystal growth inhibitor present in the liquid dosage composition ranges from about 0.1% to about 95% concentration, by weight, or from about 0.5% to about 90% concentration, by weight.
53. The method of any one of claims 43-52, wherein the effective average particle size of the nanoparticulate active agent particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
54. The method of any one of claims 43-53, wherein at least about 70%, about 90%, or about 95% of the active agent particles have a particle size less than the effective average particle size.
55. The method of any one of claims 43-54, wherein the liquid media of the liquid dosage composition is selected from the group consisting of water, safflower oil, ethanol, t-butanol, glycerin, polyethylene glycol (PEG), hexane, and glycol.
56. The method of any one of claims 43-55, wherein the at least one active agent is present in an amount selected from the group consisting of from about 99.5% to about 0.001%, from about 95% to about 0.1%, and from about 90% to about 0.5%, by weight, based on the total combined dry weight of the active agent and at least one surface stabilizer, not including other excipients.
57. The method of any one of claims 43-56, wherein the at least one surface stabilizer is present in an amount selected from the group consisting of from about 0.5% to about 99.999% by weight, from about 5.0% to about 99.9% by weight, and from about 10% to about 99.5% by weight, based on the total combined dry weight of the active agent and at least one surface stabilizer, not including other excipients.
58. The method of any one of claims 43-57, wherein the ratio of active agent to a polymeric surface modifier is selected from the group consisting of from about 20:1 to about 1:10, from about 10:1 to about 1:5, and from about 5:1 to about 1:1, by weight.
59. The method of any one of claims 43-58, comprising at least two surface stabilizers.
60. The method of claim 59, wherein the ratio of active agent to the second surface stabilizer is selected from the group consisting of from about 500:1 to about 5:1, from about 350:1 to about 10:1, and from about 100:1 to about 20:1, by weight.
61. The method of any one of claims 43-60, wherein the surface stabilizer is selected from the group consisting of an anionic surface stabilizer, a cationic surface stabilizer, a polymeric surface stabilizer, a nonionic surface stabilizer, and a zwitterionic surface stabilizer.
62. The method of claim 61, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, and random copolymers of vinyl acetate and vinyl pyrrolidone.
63. The method of claim 61, wherein the at least one cationic surface stabilizer is selected from the group consisting of a polymer, a biopolymer, a polysaccharide, a cellulosic, an alginate, a nonpolymeric compound, a phospholipid, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL™, ALKAQUAT™, alkyl pyridinium salts; amines, amine salts, amine oxides, imide azolinium salts, protonated quaternary acrylamides, methylated quaternary polymers, and cationic guar.
64. The method of any one of claims 43-63, wherein the active agent is selected from the group consisting of a crystalline phase, an amorphous phase, a semi-crystalline phase, a semi-amorphous phase, and mixtures thereof.
65. The method of any one of claims 43-64, wherein the one or more active agents have a solubility in water selected from the group consisting of less than about 30 mg/ml, less than about 20 mg/ml, less than about 10 mg/ml, and less than about 1 mg/ml, under ambient conditions.
66. The method of any one of claims 43-65, wherein the active agent comprises anti-inflammatory and analgesic properties.
67. The method of any one of claims 43-66, wherein the at least one active agent is selected from the group consisting of COX-2 inhibitors, anticancer agents, NSAIDS , proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.
68. The method of claim 67, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.
69. The method of any one of claims 43-67, wherein the active agent is selected from the group consisting of acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifene, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, and acetylsalicylate.
70. Use of a composition according to any one of claims 1 to 42 for making a medicament
71. The use of claim 70, wherein the medicament is useful in treating a condition selected from the group consisting of neoplastic diseases, breast cancer, endometrial cancer, uterine cancer, cervical cancer, prostate cancer, renal cancer, hormone replacement therapy in post-menopausal women, endometriosis, hirsutism, dysmenorrhea, uterine bleeding, HIV wasting, cancer wasting, migraine headache, cachexia, anorexia, castration, oral contraception, motion sickness, emesis related to cytotoxic drugs, gastritis, ulcers, dyspepsia, gastroenteritis, including collitis and food poisoning, inflammatory bowel disease, Crohn's disease, migraine headaches, and any other condition which is accompanied by the symptoms of nausea and vomiting.
72. The use of claim 70 or claim 71, wherein the medicament is useful in treating a condition selected from the group consisting of pain, inflammation, arthritis, cancer, kidney disease, osteoporosis, Alzheimer's disease, and familial adenomatous polyposis.
73. The use of claim 70 or claim 71, wherein the medicament is useful in treating a condition selected from the group consisting of osteoarthritis, rheumatoid arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma.

## Claims

1. A nanoparticulate liquid dosage composition, which reduces, inhibits or prevents the occurrence of crystal growth of the active agent during storage, comprising:
(a) particles of at least one active agent having an effective average particle size of less than about 2000 nm;
(b) at least one surface stabilizer; and
(c) at least one osmotically active crystal growth inhibitor;
wherein the active agent particles undergo crystal growth upon storage in the absence of the crystal growth inhibitor
and wherein the viscosity of the composition at a shear rate of 0.1 (1/S) is from 2000 mPa.s to 1 mPa.s

2. The composition of claim 1, wherein the active agent particles form needle-like crystals upon storage.

3. The composition of claim 1 or claim 2, wherein the active agent undergoes crystal growth:
(a) upon storage for 24 hours at room temperature in the absence of the crystal growth inhibitor, or,
(b) upon storage for three days at 40 °C in the absence of the crystal growth inhibitor.

4. The composition of any of claims 1 to 3, wherein:
(a) the osmotically active crystal growth inhibitor is at least partially water-soluble and does not solubilize the nanoparticulate active agent; and/or
(b) the amount of the crystal growth inhibitor present in the liquid dosage composition ranges from about 0.1% to about 95% concentration, by weight, or from about 0.5% to about 90% concentration, by weight; and/or
(c) the amount of the active agent per ml is equal to or greater than the amount of the active agent per ml of a standard conventional non-nanoparticulate liquid dosage composition of the same active agent; and/or
(d) the liquid media of the liquid dosage composition is selected from the group consisting of water, safflower oil, ethanol, t-butanol, glycerin, polyethylene glycol (PEG), hexane, and glycol.

5. The composition of any of claims 1 to 4, wherein the osmotically active crystal growth inhibitor is selected from the group consisting of glycerol, propylene glycol, mannitol, sucrose, glucose, fructose, mannose, lactose, xylitol, sorbitol, trehalose, a polysaccharide, a mono-polysaccharide, a di-polysaccharides, a sugars, a sugar alcohol, sodium chloride, potassium chloride, magnesium chloride, and an ionic salt.

6. The composition of any one of claims 1 to 5, wherein the effective average particle size of the nanoparticulate active agent particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

7. The composition of any one of claims 1 to 6, wherein:
(a) the at least one active agent is present in an amount selected from the group consisting of from about 99.5% to about 0.001%, from about 95% to about 0.1%, and from about 90% to about 0.5%, by weight, based on the total combined dry weight of the active agent and at least one surface stabilizer, not including other excipients; and/or
(b) the at least one surface stabilizer is present in an amount selected from the group consisting of from about 0.5% to about 99.999% by weight, from about 5.0% to about 99.9% by weight, and from about 10% to about 99.5% by weight, based on the total combined dry weight of the active agent and at least one surface stabilizer, not including other excipients; and/or
(c) the ratio of active agent to a polymeric surface modifier is selected from the group consisting of from about 20:1 to about 1:10, from about 10:1 to about 1:5, and from about 5:1 to about 1:1, by weight.

8. The composition of any one of claims 1 to 7, comprising at least two surface stabilizers.

9. The composition of claim 8, wherein the ratio of active agent to the second surface stabilizer is selected from the group consisting of from about 500:1 to about 5:1, from about 350:1 to about 10:1, and from about 100:1 to about 20:1, by weight.

10. The composition of any one of claims 1 to 9, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, random copolymers of vinyl acetate and vinyl pyrrolidone, cationic polymers, cationic biopolymers, cationic polysaccharides, cationic cellulosics, an alginate, a cationic nonpolymeric compound, cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOLT™, ALKAQUAT™, alkyl pyridinium salts; amines, amine salts, amine oxides, imide azolinium salts, protonated quaternary acrylamides, methylated quaternary polymers, and cationic guar.

11. The composition of any preceding claim wherein the composition is bioadhesive.

12. The composition of any one of claims 1 to 11, wherein the active agent:
(a) is selected from the group consisting of a crystalline phase, an amorphous phase, a semi-crystalline phase, a semi-amorphous phase, and mixtures thereof; and/or
(b) has a solubility in water selected from the group consisting of less than about 30 mg/ml, less than about 20 mg/ml, less than about 10 mg/ml, and less than about 1 mg/ml, under ambient conditions; and/or
(c) comprises anti-inflammatory and analgesic properties.

13. The composition of any one of claims 1 to 12, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, respiratory illness therapies associated with acquired immune deficiency syndrome, acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifene, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, and acetylsalicylate.

14. The composition of claim 13, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.

15. The composition of any one of claims 1 to 14, wherein the viscosity of the composition:
(a) at a shear rate of 0.1 (1/s), is selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s; and/or
(b) is selected from the group consisting of less than about 1/200, less than about 1/100, less than about 1/50, less than about 1/25, and less than about 1/10 of the viscosity of a standard conventional non-nanoparticulate liquid dosage composition of the same active agent at about the same concentration per ml of active agent; and/or
(c) is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional non-nanoparticulate liquid dosage composition of the same active agent at about the same concentration per ml of active agent.

16. The composition of any preceding claim, wherein:
(a) the Tₘₐₓ is selected from the group consisting of not greater than about 90%, not greater than about 80%, not greater than about 70%, not greater than about 60%, not greater than about 50%, not greater than about 30%, not greater than about 25%, not greater than about 20%, not greater than about 15%, and not greater than about 10% of the Tₘₐₓ, exhibited by a non-nanoparticulate formulation of the same active agent, administered at the same dosage; and/or
(b) the Cₘₐₓ is selected from the group consisting of at least about 10%, at least about 20%, at least about 30%, at least about 40%, at least about 50%, at least about 60%, at least about 70%, at least about 80%, at least about 90%, and at least about 100% greater than the Cₘₐₓ exhibited by a non-nanoparticulate formulation of the same active agent, administered at the same dosage; and/or
(c) the AUC is selected from the group consisting of at least about 10%, at least about 20%, at least about 30%, at least about 40%, at least about 50%, at least about 60%, at least about 70%, at least about 80%, at least about 90%, and at least about 100% greater than the AUC exhibited by a non-nanoparticulate formulation of the same active agent, administered at the same dosage.

17. The composition of any preceding claim, wherein the difference in absorption of the active agent composition of the invention, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%, or,
wherein administration of the composition to a subject in a fasted state is bioequivalent to administration of the composition to a subject in a fed state, when administered to a human.

18. The composition of any one of claims 1 to 15, wherein the composition is formulated:
(a) for administration selected from the group consisting of oral, pulmonary, rectal, ophthalmic, colonic, parenteral, intracisternal, intravaginal, intraperitoneal, local, buccal, nasal, and topical administration; and/or
(b) into a dosage form selected from the group consisting of liquid dispersions, oral suspensions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.

19. A composition according to any of claims 1-16, for use in the treatment of a condition selected from the group consisting of:
(a) neoplastic diseases, breast cancer, endometrial cancer, uterine cancer, cervical cancer, prostate cancer, renal cancer, hormone replacement therapy in post-menopausal women, endometriosis, hirsutism, dysmenorrhea, uterine bleeding, HIV wasting, cancer wasting, migraine headache, cachexia, anorexia, castration, oral contraception, motion sickness, emesis related to cytotoxic drugs, gastritis, ulcers, dyspepsia, gastroenteritis, including collitis and food poisoning, inflammatory bowel disease, Crohn's disease, migraine headaches, and any other condition which is accompanied by the symptoms of nausea and vomiting; and/or
(b) pain, inflammation, arthritis, cancer, kidney disease, osteoporosis, Alzheimer's disease, and familial adenomatous polyposis; and/or
(c) osteoarthritis, rheumatoid arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma.
